(19) Europäisches Patentamt
European Patent Office
Office européen des brevets

(11) Numéro de publication: **0 048 705**
**B1**

(12) # FASCICULE DE BREVET EUROPEEN

(45) Date de publication du fascicule du brevet: **11.07.84**

(51) Int. Cl.³: **C 07 D 211/70**, A 61 K 31/445

(21) Numéro de dépôt: **81870037.9**

(22) Date de dépôt: **16.09.81**

(54) **Nouveaux acides 2-(4-(diphénylméthylène)-1-pipéridinyl)-acétiques et leurs amides, leurs procédés de préparation et compositions thérapeutiques.**

(30) Priorité: **18.09.80 GB 8030194**

(43) Date de publication de la demande:
**31.03.82 Bulletin 82/13**

(45) Mention de la délivrance du brevet:
**11.07.84 Bulletin 84/28**

(84) Etats contractants désignés:
**AT BE CH DE FR GB IT LI LU NL SE**

(56) Documents cités:
**DE - A - 2 016 667**
**GB - A - 1 106 861**
**GB - A - 1 108 033**
**GB - A - 2 068 956**
**US - A - 2 898 339**
**US - A - 3 922 276**
**US - A - 4 251 655**

(73) Titulaire: **U C B, S.A., 326, Avenue Louise, B-1050 Bruxelles (BE)**

(72) Inventeur: **Rodriguez, Ludovic, 12, Clos Dandoy, B-1180 Bruxelles (BE)**
Inventeur: **Baltes, Eugène, 2, Avenue des Chênes, B-1640 Rhode-Saint-Genese (BE)**

(74) Mandataire: **Vanderborght, Henri et al, U C B, S.A. Département D.T.B. 33, rue d'Anderlecht, B-1620 Drogenbos (BE)**

**EP 0 048 705 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à des acides 2-[4-(diphénylméthylène)-1-pipéridinyl]acétiques, à leurs amides, à leurs sels non toxiques pharmaceutiquement acceptables ainsi qu'à des procédés pour les préparer. Elle concerne également les compositions thérapeutiques renfermant lesdits composés.

Selon le brevet belge N° 748568 au nom de la titulaire, on connaît des 4-(diphénylméthylène)-pipéridinyl substituées sur l'atome d'azote par un radical hydroxyalkyle, hydroxyalkoxyalkyle ou hydroxyalkoxyalkoxyalkyle, les radicaux alkyle et alkoxy étant à chaîne droite ou ramifiée et contenant 1 à 4 atomes de carbone. Ce brevet décrit également des essais pharmacologiques démontrant les effets bronchodilatateurs, antihistaminiques, vasodilatateurs et coronarodilatateurs de ces pipéridines, de même que leurs effets sur le système nerveux central; il n'y est toutefois pas fait mention d'une activité antiallergique. La titulaire a constaté que ces composés possèdent une toxicité non négligeable ainsi que des effets secondaires indésirables, par exemple une augmentation significative de la fréquence cardiaque (tachycardie), à des doses relativement faibles. Ces inconvénients ont amené la titulaire à ne pas envisager l'utilisation de ces composés en thérapeutique humaine courante.

La présente invention a pour but de remédier à ces inconvénients. Elle procure à cet effet de nouveaux dérivés de la pipéridine qui possèdent en plus de propriétés pharmacologiques similaires une activité antiallergique intéressante. En outre et de manière surprenante, ces nouveaux composés se distinguent avantageusement des composés décrits plus haut par leur faible toxicité et par l'absence d'effets secondaires indésirables aux doses actives.

Les nouveaux composés de la présente invention répondent à la formule générale:

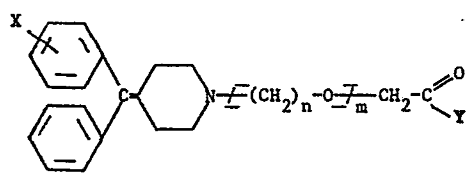
(I)

dans laquelle:

Y est un groupe $-OH$ ou un groupe $-NR_1R_2$ dans lequel $R_1$ et $R_2$ représentent indépendamment l'un de l'autre de l'hydrogène, un radical alkyle inférieur ou phényle;

X représente un atome d'hydrogène, un atome d'halogène ou un radical alkoxy inférieur;

m est égal à 0, 1 ou 2, de préférence 1 ou 2, et

n est égal à 1 ou 2, de préférence 2.

Par l'expression alkyle inférieur, on entend les radicaux hydrocarbonés aliphatiques à chaîne linéaire ou ramifiée, ayant de 1 à 4 atomes de carbone, tels que méthyle, éthyle, propyle, isopropyle et butyle. De manière analogue, l'expression alkoxy inférieur désigne des radicaux, tels que méthoxy, éthoxy, propoxy, etc., ayant de 1 à 4 atomes de carbone. L'atome d'halogène est de préférence un atome de chlore ou de fluor.

Les composés préférés conformes à l'invention sont:

- l'acide 2-[2-[2-[4-(diphénylméthylène)-1-pipéridinyl]éthoxy]éthoxy]acétique et l'amide correspondant;
- l'acide 2-[2-[4-(diphénylméthylène)-1-pipéridinyl]éthoxy]acétique;
- l'acide 2-[2-[4-[(4-fluorophényl)phénylméthylène]-1-pipéridinyl]éthoxy]acétique et l'amide correspondant;
- l'acide 2-[2-[4-[(2-chlorophényl)phénylméthylène]-1-pipéridinyl]éthoxy]acétique et l'amide correspondant, et
- le 2-[2-[4-[(4-méthoxyphényl)phénylméthylène]-1-pipéridinyl]éthoxy]acétamide.

Les composés de formule I possèdent des propriétés pharmacologiques intéressantes; ils se révèlent notamment utiles comme agents antiallergiques, antihistaminiques, bronchodilatateurs et antispasmodiques.

En plus, ils se caractérisent par le fait que leurs effets secondaires de stimulation ou de dépression du système nerveux central, couramment observés avec les antihistaminiques usuels, sont minimes. En outre, ils présentent de l'intérêt en tant qu'anesthésiques et anti-inflammatoires et ils se révèlent actifs dans l'insuffisance cérébrale ou cardiovasculaire.

*A. Procédé de préparation*

1. Les acides de formule I, dans laquelle Y = OH, sont préparés par hydrolyse, en milieu basique, d'un dérivé fonctionnel d'un acide 2-[4-(diphénylméthylène)-1-pipéridinyl]acétique, à savoir un amide ou un ester d'alkyle inférieur de formule:

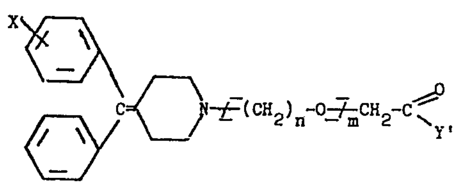
(II)

dans laquelle X, m et n ont la signification donnée plus haut et Y' représente un groupe $-NR_1R_2$, $R_1$ et $R_2$ ayant la signification donnée plus haut, ou un groupe $-OR'$ dans lequel R' représente un radical alkyle inférieur (par exemple un radical méthyle ou éthyle).

Cette hydrolyse est effectuée au moyen d'une base minérale telle que l'hydroxyde de sodium ou de potassium, en milieu aqueux ou hydroalcoolique (méthanol, éthanol, etc.), et à une température comprise entre 20° C et la température de reflux du mélange réactionnel.

Les esters de formule II dans laquelle Y' = $-OR'$, qui sont utilisés comme matière première pour la préparition des acides de l'invention selon le procédé décrit ci-dessus, peuvent être préparés par diverses méthodes, par exemple:

*E.1*

En faisant réagir une 4-(diphénylméthylène)-pipéridine de formule III avec un ω-halogénoacétate d'alkyle inférieur de formule IV, selon l'équation:

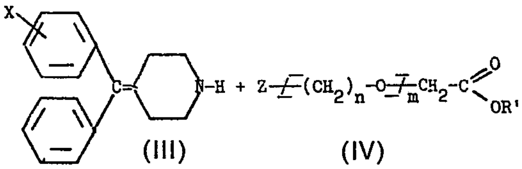

(III) (IV)

→ (II) avec Y' = OR'

dans lesquelles X, m et n ont la signification donnée plus haut, R' représente un radical alkyle inférieur et Z un atome d'halogène.

Ainsi, R' est par exemple un radical méthyle ou éthyle et Z un atome de chlore ou de brome.

Cette réaction est généralement effectuée par chauffage, entre 80 et 150°C, pendant quelques heures, dans un solvant inerte choisi parmi des alcools aliphatiques, le benzène, le toluène et le xylène et en présence d'un accepteur d'acide tel qu'une base organique tertiaire (par exemple la triéthylamine) ou une base minérale (par exemple le carbonate de sodium).

*E.2*

Dans le cas où, dans la formule II, m est égal à 1 ou 2, en faisant réagir un sel de métal alcalin d'un ω-[4-(diphénylméthylène)-1-pipéridinyl]alcanol de formule V avec un halogénoacétate d'alkyle inférieur de formule VI, selon l'équation:

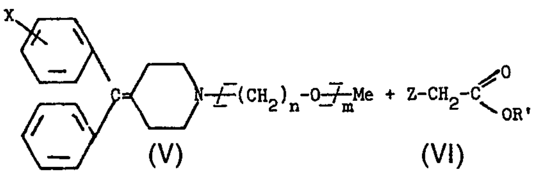

(V) (VI)

→ (II) avec Y' = OR'

dans lesquelles R', X et n ont la signification donnée plus haut, m est égal à 1 ou 2, z représente un atome d'halogène et Me représente un métal alcalin.

La réaction entre le sel métallique de formule V et l'acétate halogéné de formule VI est effectuée dans un solvant inerte, à une température comprise entre 0°C et la température de reflux du mélange réactionnel.

Le sel de métal alcalin utilisé dans cette réaction est préparé *in situ* en faisant réagir l'ω-[4-(diphénylméthylène)-1-pipéridinyl]alcanol approprié avec un hydrure de métal alcalin, généralement l'hydrure de sodium, dans un solvant inerte, tel que le toluène, le xylène ou le diméthylformamide.

Quant à la préparation des alcanols de formule V (Me = H), elle est décrite dans le brevet belge N° 748568 au nom de la titulaire.

II. Les amides de formule I ($Y = -NR_1R_2$) peuvent être préparés par diverses méthodes, à savoir:

II.1. En faisant réagir une 4-(diphénylméthylène)pipéridine de formule III avec un ω-halogénoacétamide de formule VII, selon l'équation

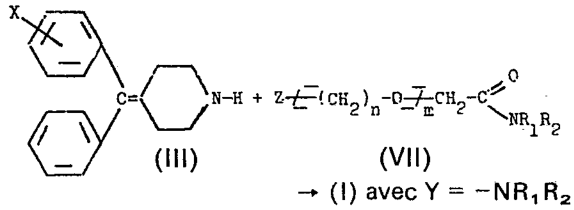

(III) (VII)

→ (I) avec $Y = -NR_1R_2$

dans lesquelles $R_1$, $R_2$, X, m et n ont la signification donnée plus haut et Z représente un atome d'halogène.

Cette réaction est généralement effectuée par chauffage, entre 80 et 150°C, pendant quelques heures, dans un solvant inerte choisi parmi des alcools aliphatiques, le benzène, le toluène et le xylène et en présence d'un accepteur d'acide tel qu'une base organique tertiaire (par exemple la triéthylamine) ou une base minérale (par exemple le carbonate de sodium).

II.2. Dans le cas où dans la formule I m est égal à 1 ou 2, en faisant réagir un sel de métal alcalin d'un ω-[4-(diphénylméthylène)-1-pipéridinyl]-alcanol de formule V avec un 2-halogénoacétamide de formule VIII, selon l'équation:

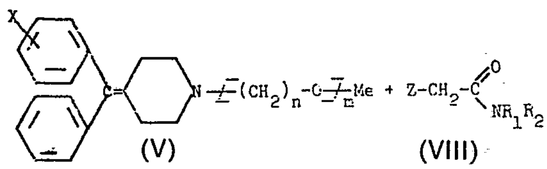

(V) (VIII)

→ (I) avec $Y = NR_1R_2$

dans lesquelles $R_1$, $R_2$, X et n ont la signification donnée plus haut, m est égal à 1 ou 2, Z représente un atome d'halogène et Me un métal alcalin.

La réaction entre le sel métallique de formule V et l'acétamide halogéné de formule VIII est effectuée dans un solvant inerte, à une température comprise entre 0°C et la température de reflux du mélange réactionnel.

II.3. En faisant réagir un composé azoté de formule X avec un dérivé fonctionnel d'un acide 2-[4-(diphénylméthylène)-1-pipéridinyl]acétique, à savoir un halogénure ou un ester d'alkyle inférieur de formule IX, selon l'équation:

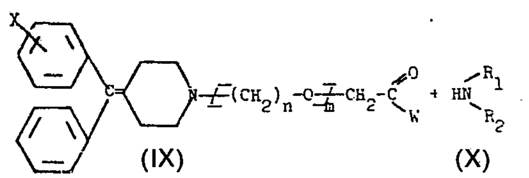

(IX) (X)

→ (I) avec $Y = -NR_1R_2$

dans lesquelles $R_1$, $R_2$, X, m et n ont la signification donnée plus haut et W représente un atome d'halogène ou un radical −OR' dans lequel R' représente un radical alkyle inférieur. L'atome d'halogène peut par exemple être du chlore ou du brome, et le radical alkyle un radical méthyle ou éthyle.

Dans le cas où W représente un atome d'halogène, on commence par préparer un acide de formule I par la méthode I décrite plus haut et on le transforme en son halogénure correspondant selon les méthodes classiques pour préparer ce

genre de composé. Ensuite, on fait réagir l'halogénure d'acide obtenu avec l'amine appropriée, dans un solvant inerte, en présence d'un accepteur d'acide, par exemple une base organique ou minérale.

Dans le cas où W représente un radical −OR', on commence par préparer un ester de formule II par l'une des méthodes décrites plus haut (E.1 ou E.2). Ensuite, on fait réagir cet ester avec l'amine appropriée, dans un solvant inerte qui peut être constitué par un excès de l'amine mise en œuvre, à une température comprise entre la température ambiante et la température de reflux du mélange réactionnel. Cette réaction peut éventuellement être effectuée en présence d'un catalyseur tel que le méthylate de sodium. Les conditions opératoires peuvent varier selon la nature et la réactivité de l'amine utilisée.

Dans le cadre de la présente invention, on entend par sels non toxiques pharmaceutiquement acceptables non seulement les sels d'addition des acides et des amides de formule I avec des acides pharmaceutiquement acceptables comme l'acide acétique, l'acide citrique, l'acide succinique, l'acide ascorbique, l'acide chlorhydrique, l'acide bromhydrique, l'acide sulfurique ou l'acide phosphorique, mais également les sels pharmaceutiquement acceptables des acides de formule I comme les sels de métaux (par exemple sodium ou potassium), les sels d'ammonium, les sels d'amine ou d'acides aminés.

Ces sels pharmaceutiquement acceptables peuvent être préparés à partir des composés de formule I par des méthodes connues en soi.

Les exemples qui suivent illustrent l'invention sans la limiter.

*Exemple 1: Préparation des esters de formule II*

1.1. *Ester éthylique de l'acide 2-[2-[2-[4-(diphénylméthylène)-1-pipéridinyl]éthoxy]éthoxy]acétique* (méthode E.2)

Une solution de 207,6 g (0,616 mol) de 2-[2-[4-(diphénylméthylène)-1-pipéridinyl]éthoxy]-éthanol dans 1,2 l de toluène anhydre est refroidie vers 10°C. On y ajoute, par portions, 17,5 g (0,729 mol) d'hydrure de sodium (à partir de 35 g d'une suspension à 50% d'hydrure de sodium dans de la paraffine et qui est lavée 3 fois avec du toluène anhydre).

Le mélange est chauffé lentement jusque vers 40°C et il est maintenu à cette température pendant 2 h. On refroidit ensuite à 0°C sous azote et on introduit, en maintenant cette température, 122 g (0,73 mol) de bromoacétate d'éthyle. La réaction est violente au début. Une fois l'addition terminée, le milieu réactionnel est maintenu à 40°C pendant 4 h, puis est ramené à la température ambiante. On filtre et on lave le précipité avec un peu de toluène. Le filtrat, après évaporation, laisse 345,6 g de résidu (ester éthylique de l'acide 2-[2-[2-[4-(diphénylméthylène)-1-pipéridinyl]éthoxy]éthoxy]acétique), qui est utilisé tel quel dans l'exemple 3.1., sans autre purification.

1.2. Le produit de l'exemple 1.1. peut aussi être obtenu dans les conditions suivantes (méthode E.1):

On chauffe pendant 20 h, entre 90 et 110°C, un mélange de 24,93 g de 4-(diphénylméthylène)-pipéridine, de 32 g de [2-(2-chloréthoxy)-éthoxy]acétate d'éthyle et de 18 g de carbonate de sodium anhydre, dans 80 ml de xylène. On filtre le précipité formé. Le filtrat est extrait par une solution diluée d'acide chlorhydrique et la phase aqueuse, après alcalinisation par une solution concentrée d'hydroxyde de sodium, est extraite au benzène. La phase benzénique est évaporée sous vide et le résidu obtenu (ester) est utilisé tel quel dans l'exemple 3.2. sans autre purification.

Le [2-(2-chloréthoxy)éthoxy]acétate d'éthyle utilisé dans cette synthèse a été préparé de la manière suivante:

On sature à froid, au moyen d'acide chlorhydrique gazeux, une solution de 100 g de [2-(2-chloréthoxy)éthoxy]acétonitrile dans 500 ml d'éthanol. On chauffe le mélange à reflux pendant 5 h, puis on le distille.

Rendement: 81,2%; P.E.: 146-148°C/20 mbar.

1.3. *Ester éthylique de l'acide 2-[2-[4-(diphénylméthylène)-1-pipéridinyl]éthoxy]acétique*

Cet ester a été préparé selon la méthode décrite dans l'exemple 1.2. à partir de (2-chloréthoxy)-acétate d'éthyle et de 4-(diphénylméthylène)-pipéridine. Il n'a pas été isolé et il est utilisé tel quel, sans autre purification, pour préparer l'acide correspondant (voir exemple 3.3.).

*Exemple 2: Préparation des amides de formule I*

2.1. *2-[2-[2-[4-(diphénylméthylène)-1-pipéridinyl]éthoxy]éthoxy]acétamide* (méthode II.1.)

On chauffe pendant 20 h, entre 90 et 110°C, un mélange de 29,9 g de 4-(diphénylméthylène)-pipéridine, de 36,3 g de 2-[2-(2-chloréthoxy)-éthoxy]acétamide et de 18 g de carbonate de sodium dans 80 ml de xylène. On ajoute ensuite 80 ml de benzène; on filtre le précipité et on extrait la couche organique avec une solution diluée d'acide chlorhydrique (20 ml d'acide chlorhydrique concentré +80 ml d'eau). Après l'addition de 30 ml d'une solution concentrée d'hydroxyde de sodium et extraction au benzène, on lave la solution benzénique, on la sèche sur du carbonate de potassium et on évapore le benzène sous vide. Le 2-[2-[2-[4-(diphénylméthylène)-1-pipéridinyl]éthoxy]éthoxy]acétamide obtenu est utilisé tel quel pour préparer l'acide correspondant (voir exemple 3.4.). Le 2-[2-(2-chloréthoxy)éthoxy]acétamide utilisé dans cette synthèse a été préparé selon le procédé décrit dans le brevet britannique No 1357547.

Rendement: 77%; P.F.: 51-53°C.

2.2. *Chlorhydrate de 2-[2-[2-[4-(diphénylméthylène)-1-pipéridinyl]éthoxy]éthoxy]acétamide* (méthode II.3.)

40 g de l'ester obtenu à l'exemple 1.1. sont dissous dans 400 ml de méthanol.

On y fait ensuite passer de l'ammoniac pendant une nuit, à température ambiante. La solution est évaporée à sec et le résidu est redissous dans de l'acétate d'éthyle. La solution est filtrée sur norite et évaporée à sec. Le résidu est dissous dans de l'éther éthylique auquel on ajoute la quantité stœchiométrique d'une solution éthérée d'acide chlorhydrique pour transformer la base en chlorhydrate. Celui-ci est recristallisé successivement dans de l'acétone et dans de l'acétonitrile. On obtient finalement 11,5 g de chlorhydrate de 2-[2-[2-[4-(diphénylméthylène)-1-pipéridinyl]éthoxy]éthoxy]acétamide.

P.F.: 145-146°C ; rendement: 28%.

*Analyse* pour $C_{24}H_{30}N_2O_3 \cdot HCl$ en %:

Calculé: C 66,9 H 7,20 N 6,50 Cl 8,25%

Trouvé: C 66,5 H 7,21 N 6,17 Cl 8,08%

2.3. Les produits suivants ont été préparés selon la méthode de l'exemple 2.1.:

*2-[4-(Diphénylméthylène)-1-pipéridinyl]-acétamide*

Rendement: 40%; P.F.: 220°C.

*Analyse* pour $C_{20}H_{22}N_2O$ en %:

Calculé: C 78,40 H 7,24 N 9,14%

Trouvé: C 77,64 H 7,36 N 8,90%

*Chlorhydrate de 2-[4-[(4-chlorophényl)-phénylméthylène]-1-pipéridinyl]acétamide*

Rendement: 77%; P.F.: 221-223°C.

*Analyse* pour $C_{20}H_{21}ClN_2O \cdot HCl$ en %:

Calculé: C 63,66 H 5,87 N 7,42
$Cl^{tot.}$ 18,79%

Trouvé: C 63,38 H 6,13 N 7,63
$Cl^{tot.}$ 18,76%

*Chlorhydrate de 2-[2-[4-[(4-chlorophényl)-phénylméthylène]-1-pipéridinyl]éthoxy]acétamide*

Rendement: 65%; P.F.: 166-169°C.

*Analyse* pour $C_{22}H_{25}ClN_2O_2 \cdot HCl$ en %:

Calculé: C 62,74 H 6,22 N 6,65
$Cl^-$ 8,42 $Cl^{tot.}$ 16,83%

Trouvé: C 61,22 H 6,40 N 6,47
$Cl^-$ 8,71 $Cl^{tot.}$ 16,51%

*2-[2-[2-[4-[(4-Chlorophényl)phénylméthylène]-1-pipéridinyl]éthoxy]éthoxy]acétamide*

Rendement: 93%.

*Analyse* pour $C_{24}H_{29}ClN_2O_3$ en %:

Calculé: N 6,53 Cl 8,63%

Trouvé: N 5,59 Cl 8,74%

Spectre de masse: ion moléculaire $M^+$ à m/e = 428.

*Chlorhydrate de 2-[2-[4-[(2-chlorophényl)-phénylméthylène]-1-pipéridinyl]éthoxy]acétamide*

Rendement: 86%; P.F.: 240-241°C.

*Analyse* pour $C_{22}H_{25}ClN_2O_2 \cdot HCl$ en %:

Calculé: C 62,70 H 6,21 N 6,64
$Cl^-$ 8,41 $Cl^{tot.}$ 16,82%

Trouvé: C 62,56 H 6,29 N 6,52
$Cl^-$ 8,17 $Cl^{tot.}$ 16,79%

*2-[2-[4-[(4-Fluorophényl)phénylméthylène-1-pipéridinyl]éthoxy]acétamide*

Rendement: 65%; P.F.: 118-119°C.

*Analyse* pour $C_{22}H_{25}FN_2O_2$ en %:

Calculé: C 71,71 H 6,84 N 7,60%

Trouvé: C 71,66 H 6,93 N 7,53%

*Chlorhydrate de 2-[2-[4-[(4-méthoxyphényl)-phénylméthylène]-1-pipéridinyl]éthoxy]acétamide.*

Rendement: 47%; P.F.: 196-198°C.

*Analyse* pour $C_{23}H_{28}N_2O_3 \cdot HCl$ en %:

Calculé: C 66,25 H 7,01 N 6,71
$Cl^-$ 8,50%

Trouvé: C 65,71 H 7,29 N 6,70
$Cl^-$ 8,40%

2.4. *Chlorhydrate de 2-[2-[2-[4-(diphénylméthylène)-1-pipéridinyl]éthoxy]éthoxy]-N-phénylacétamide* (méthode II.3.)

A une suspension de 4,4 g du chlorhydrate de l'acide obtenu à l'exemple 3.1. dans 100 ml de benzène anhydre, on ajoute une solution de 0,85 ml de chlorure de thionyle dans 10 ml de benzène. On chauffe le mélange à 80°C pendant 5 h. Ensuite, on l'évapore à sec et on obtient 5,4 g de résidu. Celui-ci est repris par 100 ml de chloroforme et on y ajoute successivement une solution de 1,01 g de triéthylamine dans 10 ml de chloroforme et une solution de 0,93 g d'aniline dans 10 ml de chloroforme, puis, goutte à goutte, une solution de 1,01 g de triéthylamine dans 25 ml de chloroforme. On agite pendant 20 h à température ambiante, puis on lave deux fois le milieu réactionnel avec de l'eau.

La phase organique est séchée sur du sulfate de sodium, décolorée par filtration sur norite, puis évaporée à sec.

Le résidu est transformé en chlorhydrate par cristallisation dans un mélange acétone/éther auquel on ajoute la quantité stœchiométrique d'une solution éthanolique d'acide chlorhydrique. On isole ainsi 1,95 g de chlorhydrate de 2-[2-[2-[4-(diphénylméthylène)-1-pipéridinyl]éthoxy]-éthoxy]-N-phénylacétamide.

Rendement: 38%; P.F.: 133-134°C.

*Analyse* pour $C_{30}H_{34}N_2O_3 \cdot HCl$ en %:

Calculé: C 71,07 H 6,91 N 5,53%

Trouvé: C 70,90 H 7,03 N 5,29%

*Exemple 3: Préparation des acides de formule I* (méthode I)

3.1. *Chlorhydrate de l'acide 2-[2-[2-[4-(diphénylméthylène)-1-pipéridinyl]éthoxy]éthoxy]acétique.*

345,6 g de l'ester obtenu à l'exemple 1.1. sont dissous dans 0,9 l d'éthanol. Cette solution est

ajoutée, à 20°C, à une solution contenant 288 g d'hydroxyde de potassium dans 1,5 l d'eau. On distille l'alcool jusqu'à la température de 98°C (t° de la colonne). Le milieu réactionnel est ensuite évaporé à sec et le résidu est agité dans du toluène anhydre. On obtient deux phases et un peu de solide. La phase toluénique est isolée et évaporée. On obtient 291,5 g de résidu. Celui-ci est dissous dans 1,5 l d'alcool isopropylique auquel on ajoute, à 20°C, 137 ml d'une solution alcoolique d'acide chlorhydrique (4,91N). On évapore à sec, et le résidu est recristallisé dans de l'acétonitrile, puis dans l'alcool isopropylique. On obtient ainsi 104 g de chlorhydrate de l'acide 2-[2-[2-[4-(diphénylméthylène)-1-pipéridinyl]éthoxy]éthoxy]acétique.

Rendement: 39%; P.F.: 139-140°C.

*Analyse* pour $C_{24}H_{29}NO_4 \cdot HCl$ en %:
Calculé: C 66,7 H 6,95 N 3,24 $Cl^-$ 8,40%
Trouvé: C 66,62 H 6,95 N 3,27 $Cl^-$ 8,21%

3.2. L'acide obtenu à l'exemple 3.1. peut aussi être préparé à partir de l'ester correspondant préparé à l'exemple 1.2. Le résidu (ester), obtenu à l'exemple 1.2. après l'évaporation sous vide, est dissous dans 100 ml d'éthanol et 23 ml d'hydroxyde de sodium (3,95N). Après 1½ h de chauffage à reflux, le mélange réactionnel est neutralisé par 20,7 ml d'acide chlorhydrique (4,38N) et l'éthanol est évaporé sous vide. Le résidu est extrait au dichlorométhane et la phase organique, après dessiccation sur du sulfate de sodium, est évaporée à sec. Le résidu est agité pendant 1 h dans 100 ml d'acétate d'éthyle et on laisse cristalliser.

On obtient 13,4 g d'acide 2-[2-[2-[4-(diphénylméthylène)-1-pipéridinyl]éthoxy]éthoxy]acétique.

Rendement: 67,5%; P.F.: 120-123°C.

3.3. Le produit suivant a été préparé selon la méthode décrite à l'exemple 3.2.:

*Chlorhydrate de l'acide 2-[2-[4-(diphénylméthylène)-1-pipéridinyl]éthoxy]acétique*

A partir de l'ester obtenu à l'exemple 1.3.
Rendement: 71%; P.F.: 193-194°C.

*Analyse* pour $C_{22}H_{25}NO_3 \cdot HCl$ en %:
Calculé: C 68,12 H 6,75 N 3,61 $Cl^-$ 9,14%
Trouvé: C 67,37 H 6,78 N 3,56 $Cl^-$ 8,93%

3.4. *Acide 2-[2-[2-[4-(diphénylméthylène)-1-pipéridinyl]éthoxy]éthoxy]acétique.*

Le résidu (amide) obtenu à l'exemple 2.1. est dissous dans 120 ml d'éthanol. On ajoute 60 ml d'hydroxyde de sodium aqueux (3,95N) et on chauffe le mélange à l'ébullition pendant 1½ h. Après refroidissement, on neutralise par addition de 54 ml d'acide chlorhydrique (4,38N) et on évapore l'éthanol sous vide. La solution résultante est extraite au dichlorométhane, séchée sur du sulfate de sodium et évaporée à sec. Le résidu est agité avec 150 ml d'acétate d'éthyle et on laisse cristalliser. On obtient 38 g d'acide 2-[2-[2-[4-(diphénylméthylène)-1-pipéridinyl]éthoxy]éthoxy]acétique. Le produit obtenu est identique à celui préparé à l'exemple 3.2.

Rendement: 80%; P.F.: 121-123°C.

*Analyse* pour $C_{24}H_{29}NO_4$ en %:
Calculé: C 72,88 H 7,39 N 3,54%
Trouvé: C 71,42 H 7,45 N 3,57%

3.5. Les produits suivants ont été préparés par la méthode de l'exemple 3.4., par hydrolyse de l'amide correspondant préparé à l'exemple 2.3.

*Acide 2-[4-[(4-chlorophényl)phénylméthylène]-1-pipéridinyl]acétique*
Rendement: 71%; P.F.: 190-192°C.

*Analyse* pour $C_{20}H_{20}ClNO_2$ en %:
Calculé: C 70,27 H 5,90 N 4,09 Cl 10,37%
Trouvé: C 69,42 H 5,98 N 4,08 Cl 10,99%

*Chlorhydrate de l'acide 2-[2-[4-[(4-chlorophényl)phénylméthylène]-1-pipéridinyl]éthoxy]acétique*
Rendement: 70%; P.F.: 166-168°C.

*Analyse* pour $C_{22}H_{24}ClNO_3 \cdot HCl$ en %:
Calculé: C 62,56 H 5,96 N 3,31 $Cl^-$ 8,39 $Cl^{tot.}$ 16,78%
Trouvé: C 62,51 H 6,09 N 3,43 $Cl^-$ 8,39 $Cl^{tot.}$ 16,54%

*Acide 2-[2-[2-[4-[(4-chlorophényl)phénylméthylène]-1-pipéridinyl]éthoxy]éthoxy]acétique*
Rendement: 80%; P.F.: 112-115°C.

*Analyse* pour $C_{24}H_{28}ClNO_4$ en %:
Calculé: C 67,04 H 6,56 N 3,25 Cl 8,55%
Trouvé: C 66,13 H 6,55 N 2,82 Cl 8,74%

Spectre de masse: ion moléculaire $M^+$ à m/e = 429.

Le chlorhydrate correspondant fond à 105-108°C (décomposition).

*Analyse* pour $C_{24}H_{28}ClNO_4 \cdot HCl$ en %:
Calculé: $Cl^-$ 7,60 $Cl^{tot.}$ 15,20%
Trouvé: $Cl^-$ 6,99 $Cl^{tot.}$ 15,20%

*Chlorhydrate de l'acide 2-[2-[4-[(2-chlorophényl)phénylméthylène]-1-pipéridinyl]éthoxy]acétique*
Rendement: 94%; P.F.: 198-200°C.

*Analyse* pour $C_{22}H_{24}ClNO_3 \cdot HCl$ en %:
Calculé: C 62,56 H 5,96 N 3,31 $Cl^-$ 8,39 $Cl^{tot.}$ 16,78%
Trouvé: C 62,51 H 5,80 N 3,30 $Cl^-$ 8,35 $Cl^{tot.}$ 17,36%

*Acide 2-[2-[4-[(4-fluorophényl)phénylméthylène]-1-pipéridinyl]éthoxy]acétique*
Rendement: 96%; P.F.: 72-74°C.

*Analyse* pour $C_{22}H_{24}FNO_3$ en %:
Calculé: C 71,52 H 6,52 N 3,74%
Trouvé: C 71,05 H 6,05 N 3,90%

Spectre de masse: ion moléculaire $M^+$ à m/e = 369.

*Chlorhydrate de l'acide 2-[2-[4-[(4-méthoxyphényl)phénylméthylène]-1-pipéridinyl]éthoxy]acétique*

Rendement: 65%; P.F.: 184-187°C (décomposition).

*Analyse* pour $C_{28}H_{27}NO_4 \cdot HCl$ en %:
Calculé: C 66,17 H 6,76 N 3,35 $Cl^-$ 8,49%
Trouvé: C 66,27 H 6,79 N 3,51 $Cl^-$ 8,44%

*B. Pharmacologie*

Les produits suivants de la présente invention ont été soumis à des essais pharmacologiques dont les résultats sont reproduits ci-après:

– chlorhydrate de l'acide 2-[2-[2-[4-(diphénylméthylène)-1-pipéridinyl]éthoxy]éthoxy]acétique (produit A, préparé à l'exemple 3.1.);

– chlorhydrate de l'acide 2-[2-[4-(diphénylméthylène)-1-pipéridinyl]éthoxy]acétique (produit B, préparé à l'exemple 3.3.);

– chlorhydrate de 2-[2-[2-[4-(diphénylméthylène)-1-pipéridinyl]éthoxy]éthoxy]acétamide (produit C, préparé à l'exemple 2.2.);

– chlorhydrate de 2-[2-[4-[(4-chlorophényl)phénylméthylène]-1-pipéridinyl]éthoxy]acétamide (produit D, préparé à l'exemple 2.3.);

– 2-[2-[2-[4-[(4-chlorophényl)phénylméthylène]-1-pipéridinyl]éthoxy]éthoxy]acétamide (produit E, préparé à l'exemple 2.3.);

– chlorhydrate de 2-[2-[4-[(2-chlorophényl)phénylméthylène]-1-pipéridinyl]éthoxy]acétamide (produit F, préparé à l'exemple 2.3.);

– 2-[2-[4-[(4-fluorophényl)phénylméthylène]-1-pipéridinyl]éthoxy]acétamide (produit G, préparé à l'exemple 2.3.);

– chlorhydrate de 2-[2-[4-[(4-méthoxyphényl)phénylméthylène]-1-pipéridinyl]éthoxy]acétamide (produit H, préparé à l'exemple 2.3.);

– chlorhydrate de l'acide 2-[2-[4-[(4-chlorophényl)phénylméthylène]-1-pipéridinyl]éthoxy]acétique (produit I, préparé à l'exemple 3.5.);

– chlorhydrate de l'acide 2-[2-[2-[4-[(4-chlorophényl)phénylméthylène]-1-pipéridinyl]éthoxy]éthoxy]acétique (produit J, préparé à l'exemple 3.5.);

– chlorhydrate de l'acide 2-[2-[4-[(2-chlorophényl)phénylméthylène]-1-pipéridinyl]éthoxy]acétique (produit K, préparé à l'exemple 3.5.);

– acide 2-[2-[4-[(4-fluorophényl)phénylméthylène]-1-pipéridinyl]éthoxy]acétique (produit L, préparé à l'exemple 3.5.);

– chlorhydrate de l'acide 2-[2-[4-[(4-méthoxyphényl)phénylméthylène]-1-pipéridinyl]éthoxy]acétique (produit M, préparé à l'exemple 3.5.);

– chlorhydrate de 2-[2-[2-[4-(diphénylméthylène)-1-pipéridinyl]éthoxy]éthoxy]-N-phénylacétamide (produit N, préparé à l'exemple 2.4.).

*1. Activité antiallergique*

Cette activité a été déterminée, chez le rat, au moyen du test Passive Cutaneous Anaphylaxis (PCA) (voir J. Goose et A.M.J.N. Blair, «Immunology», *16* (1969), 749-760 et U. Martin et D. Roemer, «Arzneimittel-Forschung», *28* (5) (1978), 770-782).

On utilise des rats femelles dont les flancs ont été partiellement rasés. Dans la zone ainsi rasée, on injecte par voie intradermique, pour sensibiliser passivement les animaux, 0,05 ml de sérum IGE antiovalbumine à une dilution telle que, lors du test PCA, une tache nette d'une superficie d'environ 100 mm² apparaît à l'endroit de l'injection.

72 h après l'injection, on administre, par voie intraveineuse, 0,25 ml d'une solution d'allergène renfermant un colorant (5 mg d'ovalbumine et 6 mg de bleu Evans dans 0,25 ml d'une solution de chlorure de sodium à 0,9%). A l'endroit de l'injection intradermique, on voit aparaître une tache bleue nette, dont on mesure la surface.

Pour tester l'activité des produits de l'invention, on opère de la même manière; toutefois

– le produit à tester est administré, *per os*, 72 h après l'injection du sérum;

– 15 min après cette administration, on injecte, par voie intraveineuse, 0,25 ml de la solution d'allergène;

– 30 min après l'administration de l'allergène, on mesure la surface de la tache bleue.

Dans le tableau ci-dessous, on reproduit les doses immunologiques (DI 50, µmol/kg) qui provoquent, en moyenne sur l'ensemble des animaux soumis au test, une réduction de 50% de la superficie de la tache colorée.

De ce tableau, il ressort que, contrairement au cromoglycate de sodium, produit dont l'activité antiasthmatique par voie intraveineuse est bien connue, les produits de l'invention sont actifs *per os*.

| *Produits* | *DI 50* per os (µmol/kg) |
|---|---|
| Cromoglycate de sodium | inactif |
| A | 133 |
| B | 39 |
| C | 76 |
| D | 67,4 |
| E | 34 |
| F | 66 |
| G | 5,5 |
| H | 31,4 |
| I | 100 |
| J | 100 |
| K | 100 |

| *Produits* Cromoglycate de sodium | *DI 50* per os (µmol/kg) inactif |
|---|---|
| L | 34 |
| M | 208 |
| N | 15,2 |

*2. Activité spasmolytique et antihistaminique*

Ces activités ont été mesurées, chez le cobaye, par la méthode de H. Konzett et R. Roessler [Naunyn-Schmiedebergs «Arch. exp. Path. Pharmakol.», *195* (1940), 71-74] et comparées à celle de la théophylline.

Le cobaye anesthésié et curarisé est ventilé artificiellement. La pression endotrachéale est enregistrée. Des spasmes bronchiques répétés sont induits par des injections intraveineuses successives et progressives, respectivement d'acétylcholine, d'histamine et de sérotonine.

Les substances à tester sont également administrées par voie intraveineuse.

Dans le tableau ci-dessous figurent les doses de produits (µg/kg) qui inhibent de 50%, en moyenne sur l'ensemble des animaux, les bronchospasmes induits:

| *Produits* | *Sérotonine* | *Histamine* | *Acétylcholine* |
|---|---|---|---|
| Théophylline | 2560 | 2650 | 4130 |
| A | 41 | 47 | 1030 |
| B | 206 | 37 | >82500 |
| C | 9 | 31 | 470 |
| E | 85 | 348 | > 4285 |
| F | 55 | 551 | > 1347 |
| G | 23 | 93 | >11776 |
| H | 180 | 26 | >41650 |
| I | 1177 | 413 | 13500 |
| J | 796 | 283 | >46600 |
| K | 430 | 104 | 39457 |
| L | 198 | 58 | >11810 |
| M | 893 | 181 | >41750 |
| N | 86 | 192 | 1347 |

Il ressort de ce tableau que, par rapport à la théophylline, les substances de l'invention ont une activité spasmolytique modérée envers les bronchospasmes induits par l'acétylcholine, mais remarquable vis-à-vis des bronchospasmes induits respectivement par la sérotonine et l'histamine.

Par ailleurs, ce test a mis en évidence que certains composés administrés à une dose unique possèdent une activité antihistaminique de longue durée. Ainsi, par exemple, le produit A, administré au cobaye à la dose de 1 mg/kg par voie intraveineuse, qui possède encore une activité antihistaminique de 100% après 90 min, conserve cette même activité 4 h après son injection.

*3. Activité broncholytique*

Cette activité a été évaluée, chez le chien, au moyen du test Chien Pilocarpine [J. Mead et J.L. Whittenberger, «J. appl. Physiol.», *5* (1953), 779-796 et J. Lulling *et al.*, «Med. Pharmacol. Exp.», *16* (1967), 481-495].

Le chien anesthésié et curarisé est ventilé artificiellement. La pression endotrachéale est enregistrée. Un spasme respiratoire constant est induit par une perfusion intraveineuse continue de pilocarpine. Les substances à tester sont également administrées par voie intraveineuse.

A la dose de 320 µg/kg, le produit A réduit de 50%, en moyenne sur l'ensemble des animaux soumis au test, l'intensité du spasme induit.

Comparativement, la théophylline injectée à une dose 10 fois supérieure de 3200 µg/kg ne réduit que de 35% l'intensité du spasme induit.

Il apparaît donc que le produit A de l'invention a une activité broncholytique nettement supérieure à celle de la théophylline.

*4. Comportement général de la souris (test d'Irwin)*

Ce comportement a été étudié par le test d'Irwin [S. Irwin, «General philosophy and methodology of screening: a multidimensional approach». Gordon Research Conference on Medicinal Chemistry, Aug. 3-7 (1959) at Colby Junior College – New London].

Des doses progressives du produit soumis au test sont administrées, par voie intrapéritonéale, à des groupes de trois souris mâles (pesant de 18 à 22 g) et le comportement général des animaux est observé selon les critères classiques. Comme substances de référence, on utilise les produits suivants:

– hydroxyzine: 1-(p-chloroalphaphénylbenzyl)-4-(2-hydroxyéthoxyéthyl)pipérazine;

– oxazepam: 7-chloro-1,3-dihydro-3-hydroxy-5-phényl-2H-1,4-benzodiazépine-2-one.

Dans le tableau ci-dessous, on donne les doses (mg/kg) qui induisent les premières manifestations de tranquillisation chez les animaux.

| *Produits* | *Dose tranquillisante* (mg/kg) |
|---|---|
| A | 130 |
| B | 105 |
| F | pas d'effet sédatif |
| I | 76 |
| J | 47 |
| K | 126 |
| L | 110 |
| M | 125 |
| Hydroxyzine | 27 |
| Oxazepam | 2,6 |

Il ressort de ce tableau que les produits de l'invention ont peu d'effet sédatif comparativement aux substances de référence (doses nettement inférieures).

Par ailleurs, dans ce test, la toxicité des produits de l'invention apparaît comme étant très faible. Cette toxicité en administration par voie intrapéritonéale chez la souris (dose qui amène la mort de deux animaux sur trois) est donnée dans le tableau suivant:

| *Produits* | *Toxicité* (mg/kg) |
|---|---|
| A | 432 |
| B | 233 |
| C | 258 |
| D | 126 |
| E | 128 |
| F | 421 |
| G | 368 |
| H | 416 |
| I | 844 |
| J | 140 |
| K | 422 |
| L | 369 |
| M | 418 |

*5. Toxicité*

Les composés de l'invention sont peu toxiques; leur toxicité DL 50 (mg/kg) a été déterminée, en administration *per os*, chez le rat et la souris.

Ainsi, pour le produit A, cette toxicité est respectivement de 1903 mg/kg chez le rat et de 959 mg/kg chez la souris.

*6. Posologie et administration*

Les compositions pharmaceutiques de l'invention peuvent être administrées par voie orale, parentérale ou rectale. Elles peuvent aussi être utilisées par instillation nasale (aérosols) ou sous forme d'onguents ou de crèmes. Les compositions pharmaceutiques utilisables pour l'administration orale peuvent être solides ou liquides, par exemple sous forme de comprimés (enrobés ou non), de pilules, de dragées, de capsules en gélatine, de solutions, de sirops, etc. De même, les compositions utilisables pour l'administration par voie parentérale sont les formes pharmaceutiquement connues pour ce genre d'administration, par exemple des solutions, suspensions ou émulsions aqueuses ou huileuses.

Pour l'administration par voie rectale, les compositions de l'invention se présentent généralement sous forme de suppositoires.

Les formes pharmaceutiques telles que solutions injectables, suspensions injectables, comprimés, gouttes, suppositoires, etc., sont préparées selon les méthodes couramment utilisées par les pharmaciens. On mélange les composés de l'invention avec un véhicule solide ou liquide, non toxique, pharmaceutiquement acceptable et éventuellement avec un agent dispersant, un agent désintégrant, un agent stabilisant, etc. On peut y ajouter, le cas échéant, des préservatifs, des agents édulcorants, des agents colorants, etc. Le pourcentage de produit actif dans les compositions pharmaceutiques peut varier dans des limites très larges, selon le patient et le mode d'administration, en particulier selon la fréquence d'administration.

Quant à la posologie, elle peut varier dans une gamme étendue de doses unitaires, par exemple de 0,5 à 500 mg de produit actif. Ainsi, on peut obtenir les effets désirés en administrant, par voie intraveineuse, une dose unitaire de 30 mg ou, *per os*, une gélule de 100 mg, 1 à 2 fois/d.

A titre d'exemples non limitatifs de compositions contenant les composés de l'invention, on donne ci-après:

a) Un exemple de formule pour ampoule utilisable par voie intraveineuse:

| | |
|---|---|
| Produit A | 50 mg |
| Chlorure de sodium | 90 mg |
| Acétate de sodium | 20 mg |
| Hydroxyde de sodium pour amener le pH à 5,5 | |
| Eau distillée | 10 ml |

De même, on peut utiliser les ampoules de 10 ml renfermant respectivement 4, 20, 30 ou 200 mg de produit actif.

b) Un exemple d'une gélule utilisable *per os*:

| | |
|---|---|
| Produit A | 100 mg |
| Lactose | 344 mg |
| Cellulose (Avicel) | 50 mg |
| Dioxyde de silicium (aérosil) | 1 mg |
| Stéarate de magnésium | 5 mg |

De même, on peut utiliser des gélules à 10 ou à 50 mg de produit actif.

**Revendications** pour les Etats contractants: BE, CH, DE, FR, GB, IT, LI, LU, NL, SE

1. Acides 2-[4-(diphénylméthylène)-1-pipéridinyl]acétiques et leurs amides répondant à la formule générale:

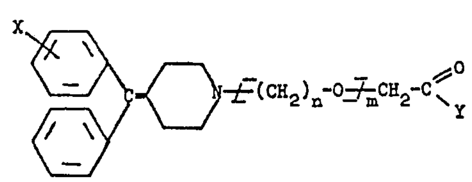

(I)

dans laquelle

Y est un groupe $-OH$ ou un groupe $-NR_1R_2$

dans lequel $R_1$ et $R_2$ représentent indépendamment l'un de l'autre de l'hydrogène, un radical alkyle ayant de 1 à 4 atomes de carbone ou phényle,

X représente un atome d'hydrogène, un atome d'halogène ou un radical alkoxy ayant de 1 à 4 atomes de carbone,

m est égal à 0, 1 ou 2, et

n est égal à 1 ou 2,

ainsi que leurs sels non toxiques pharmaceutiquement acceptables.

2. Acide 2-[2-[2-[4-(diphénylméthylène)-1-pipéridinyl]éthoxy]éthoxy]acétique et son chlorhydrate.

3. Acide 2-[2-[4-(diphénylméthylène)-1-pipéridinyl]éthoxy]acétique et son chlorhydrate.

4. 2-[2-[2-[4-(Diphénylméthylène)-1-pipéridinyl]éthoxy]éthoxy]acétamide et son chlorhydrate.

5. Acide 2-[2-[4-[(4-fluorophényl)phénylméthylène]-1-pipéridinyl]éthoxy]acétique.

6. 2-[2-[4-[(4-Fluorophényl)phénylméthylène]-1-pipéridinyl]éthoxy]acétamide.

7. 2-[2-[4-[(4-Méthoxyphényl)phénylméthylène]-1-pipéridinyl]éthoxy]acétamide et son chlorhydrate.

8. 2-[2-[4-[(2-chlorophényl)phénylméthylène]-1-pipéridinyl]éthoxy]acétamide et son chlorhydrate.

9. Procédé de préparation d'acides 2-[4-(diphénylméthylène)-1-pipéridinyl]acétiques, de leurs amides et de leurs sels tels que définis dans la revendication 1, caractérisé en ce que:

a) pour préparer les acides 2-[4-(diphénylméthylène)-1-pipéridinyl]acétiques répondant à la formule générale I donnée à la revendication 1 et dans laquelle Y = OH, on hydrolyse, en milieu aqueux ou hydroalcoolique et par une base minérale, un dérivé fonctionnel d'un acide 2-[4-(diphénylméthylène)-1-pipéridinyl]acétique de formule:

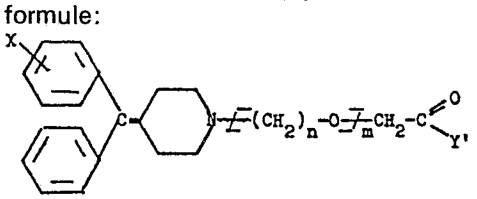
(II)

dans laquelle X, m et n ont la signification donnée à la revendication 1 et Y' représente un groupe $-NR_1R_2$, $R_1$ et $R_2$ ayant la signification donnée à la revendication 1, ou un groupe $-OR'$, dans lequel R' représente un radical alkyle ayant de 1 à 4 atomes de carbone; ou

b) pour préparer les amides de 2-[4-(diphénylméthylène)-1-pipéridinyl]acétiques répondant à la formule générale I donnée à la revendication 1 et dans laquelle $Y = -NR_1R_2$, on fait réagir, dans un solvant inerte et en présence d'un accepteur d'acide, une 4-(diphénylméthylène)-pipéridine de formule:

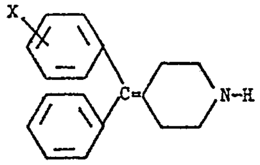
(III)

dans laquelle X a la signification donnée à la revendication 1, avec un ω-halogénoacétamide de formule:

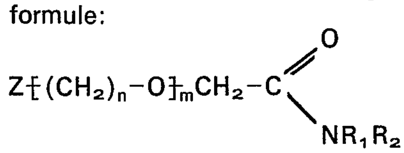
(VII)

dans laquelle $R_1$, $R_2$, m et n ont la signification donnée à la revendication 1, et Z représente un atome d'halogène; ou

c) pour préparer les amides d'acides 2-[4-(diphénylméthylène)-1-pipéridinyl]acétiques répondant à la formule générale I donnée à la revendication 1 et dans laquelle m est égal à 1 ou 2 et $Y = -NR_1R_2$, on fait réagir, dans un solvant inerte, un sel de métal alcalin d'un ω-[4-(diphénylméthylène)-1-pipéridinyl]alcanol de formule:

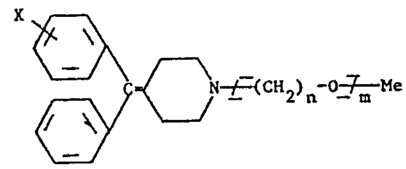
(V)

dans laquelle X et n ont la signification donnée à la revendication 1, m est égal à 1 ou 2 et Me représente un métal alcalin, avec un 2-halogénoacétamide de formule:

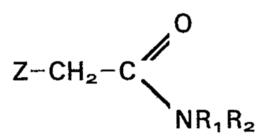
(VIII)

dans laquelle $R_1$ et $R_2$ ont la signification donnée à la revendication 1, et Z représente un atome d'halogène; ou

d) pour préparer les amides d'acides 2-[4-(diphénylméthylène)-1-pipéridinyl]acétiques répondant à la formule générale I dans laquelle $Y = -NR_1R_2$, on fait réagir, dans un solvant inerte, un composé azoté de formule:

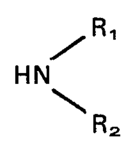
(X)

dans laquelle $R_1$ et $R_2$ ont la signification donnée à la revendication 1, avec un dérivé fonctionnel d'un acide 2-[4-(diphénylméthylène)-1-pipéridinyl]acétique de formule:

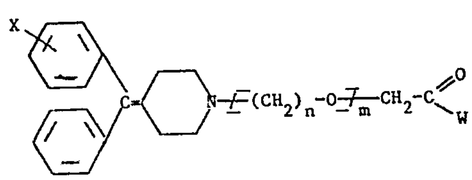
(IX)

dans laquelle X, m et n ont la signification donnée à la revendication 1, et W représente un atome d'halogène ou un groupe $-OR'$, dans lequel R' représente un radical alkyle ayant de 1 à 4 atomes de carbone; et

e) en ce que, éventuellement, on convertit les acides ou les amides d'acides 2-[4-(diphénylméthylène)-1-pipéridinyl]acétiques ainsi obtenus en leurs sels non toxiques pharmaceutiquement acceptables.

10. Compositions thérapeutiques renfermant une quantité thérapeutiquement efficace d'un composé de formule I selon la revendication 1 ou d'un sel non toxique pharmaceutiquement acceptable de celui-ci, en association avec des excipients pharmaceutiques solides ou liquides.

**Revendication** pour l'Etat contractant: AT

Procédé de préparation d'acides 2-[4-(diphénylméthylène)-1-pipéridinyl]acétiques et de leurs amides répondant à la formule générale:

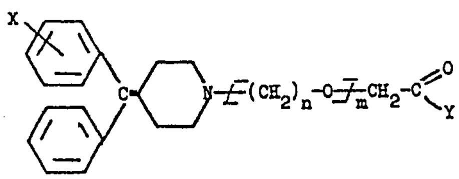

(I)

dans laquelle

Y est un groupe −OH ou un groupe $-NR_1R_2$ dans lequel $R_1$ et $R_2$ représentent indépendamment l'un de l'autre de l'hydrogène, un radical alkyle ayant de 1 à 4 atomes de carbone ou phényle,

X représente un atome d'hydrogène, un atome d'halogène ou un radical alkoxy ayant de 1 à 4 atomes de carbone,

m est égal à 0, 1 ou 2, et

n est égal à 1 ou 2

ainsi que de leurs sels non toxiques pharmaceutiquement acceptables, caractérisé en ce que:

a) pour préparer les acides 2-[4-(diphénylméthylène)-1-pipéridinyl]acétiques répondant à la formule générale (I) dans laquelle Y = OH, on hydrolyse, en milieu aqueux ou hydroalcoolique et par une base minérale, un dérivé fonctionnel d'un acide 2-[4-(diphénylméthylène)-1-pipéridinyl]-acétique de formule:

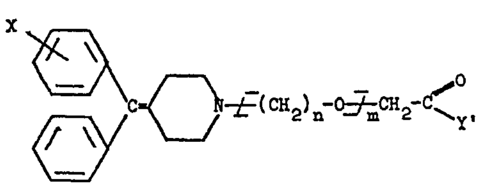

(II)

dans laquelle X, m et n ont la signification donnée ci-dessus et Y' représente un groupe $-NR_1R_2$, $R_1$ et $R_2$ ayant la signification donnée ci-dessus, ou un groupe −OR' dans lequel R' représente un radical alkyle ayant de 1 à 4 atomes de carbone; ou

b) pour préparer les amides d'acides 2-[4-(diphénylméthylène)-1-pipéridinyl]acétiques répondant à la formule générale (I) dans laquelle Y = $-NR_1R_2$, on fait réagir, dans un solvant inerte et en présence d'un accepteur d'acide, une 4-(diphénylméthylène)-1-pipéridine de formule:

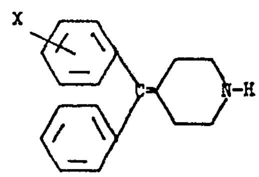

(III)

dans laquelle X a la signification donnée ci-dessus, avec un ω-halogénoacétamide de formule:

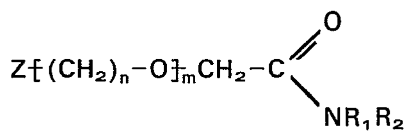

(VII)

dans laquelle $R_1$, $R_2$, m et n ont la signification donnée ci-dessus et Z représente un atome d'halogène; ou

c) pour préparer les amides d'acides 2-[4-(diphénylméthylène)-1-pipéridinyl]acétiques répondant à la formule générale (I) dans laquelle m est égal à 1 ou 2 et Y = $-NR_1R_2$, on fait réagir, dans un solvant inerte, un sel de métal alcalin d'un ω-[4-(diphénylméthylène)-1-pipéridinyl]alcanol de formule:

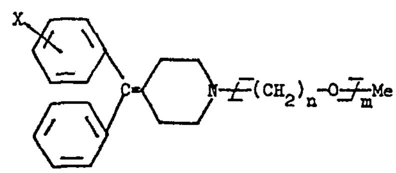

(V)

dans laquelle X et n ont la signification donnée ci-dessus, m est égal à 1 ou 2 et Me représente un métal alcalin, avec un 2-halogénoacétamide de formule:

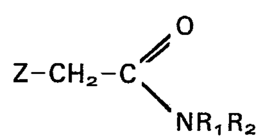

(VIII)

dans laquelle $R_1$ et $R_2$ ont la signification donnée ci-dessus, et Z représente un atome d'halogène; ou

d) pour préparer les amides d'acides 2-[4-(diphénylméthylène)-1-pipéridinyl]acétiques répondant à la formule générale (I) dans laquelle Y = $-NR_1R_2$, on fait réagir, dans un solvant inerte, un composé azoté de formule:

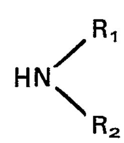

(X)

dans laquelle $R_1$ et $R_2$ ont la signification donnée ci-dessus, avec un dérivé fonctionnel d'un acide 2-[4-(diphénylméthylène)-1-pipéridinyl]acétique de formule:

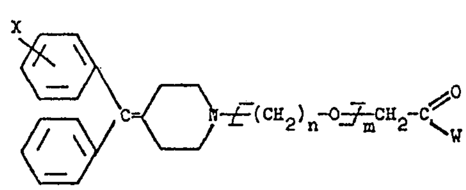

(IX)

dans laquelle X, m et n ont la signification donnée ci-dessus et W représente un atome d'halogène ou un groupe $-OR'$, dans lequel R' représente un radical alkyle ayant de 1 à 4 atomes de carbone; et

e) en ce que, éventuellement, on convertit les acides ou les amides d'acides 2-[4-diphényl-méthylène)-1-pipéridinyl]acétiques ainsi obtenus en leurs sels non toxiques pharmaceutiquement acceptables.

**Claims** for the Contracting States: BE, CH, DE, FR, GB, IT, LI, LU, NL and SE

1. 2-[4-(Diphenylmethylene)-1-piperidinyl]-acetic acids and their amides having the general formula—

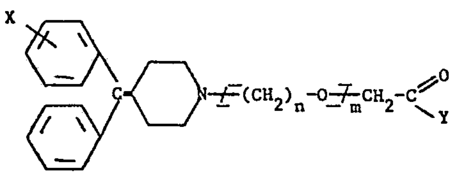

(I)

wherein

Y is an $-OH$ group or an $-NR_1R_2$ group, wherein $R_1$ and $R_2$ represent independently of each other hydrogen, an alkyl radical having 1 to 4 carbon atoms or a phenyl radical,

X represents a hydrogen atom, a halogen atom or an alkoxy radical having 1 to 4 carbon atoms,

m is 0, 1 or 2, and

n is 1 or 2,

as well as the non-toxic pharmaceutically acceptable salts thereof.

2. 2-[2-[2-[4-(Diphenylmethylene)-1-piperidinyl]ethoxy]ethoxy]acetic acid and its hydrochloride.

3. 2-[2-[4-(Diphenylmethylene)-1-piperidinyl]ethoxy]acetic acid and its hydrochloride.

4. 2-[2-[2-[4-(Diphenylmethylene)-1-piperidinyl]ethoxy]ethoxy]acetamide and its hydrochloride.

5. 2-[2-[4-[(4-Fluorophenyl)phenylmethylene]-1-piperidinyl]ethoxy]acetic acid.

6. 2-[2-[4-[(4-Fluorophenyl)phenylmethylene]-1-piperidinyl]ethoxy]acetamide.

7. 2-[2-[4-[(4-Methoxyphenyl)phenylmethylene]-1-piperidinyl]ethoxy]acetamide and its hydrochloride.

8. 2-[2-[4-[(2-Chlorophenyl)phenylmethylene]-1-piperidinyl]ethoxy]acetamide and its hydrochloride.

9. Process for the preparation of 2-[4-(diphenylmethylene)-1-piperidinyl]acetic acids, their amides and their salts as defined in Claim 1, characterised in that—

(*a*) in order to prepare the 2-[4-(diphenylmethylene)-1-piperidinyl]acetic acids having the general formula (I) given in Claim 1 and wherein Y = OH, a functional derivative of a 2-[4-(diphenylmethylene)-1-piperidinyl]acetic acid of the formula—

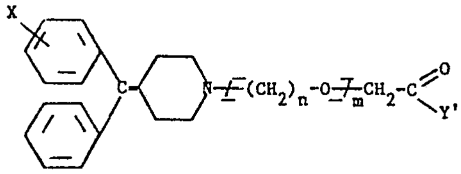

(II)

wherein X, m and n have the meaning given in Claim 1 and Y' represents an $-NR_1R_2$ group, $R_1$ and $R_2$ having the meaning given in Claim 1, or an $-OR'$ group, wherein R' represents an alkyl radical having 1 to 4 carbon atoms, is hydrolysed, in an aqueous or hydroalcoholic medium, and with an inorganic base; or

(*b*) in order to prepare the amides of 2-[4-(diphenylmethylene)-1-piperidinyl]acetic acids having the general formula (I) given in Claim 1 and wherein $Y = -NR_1R_2$, a 4-(diphenylmethylene)-piperidine of the formula—

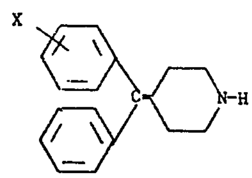

(III)

wherein X has the meaning given in Claim 1, is reacted, in an inert solvent and in the presence of an acid acceptor, with an ω-haloacetamide of the formula—

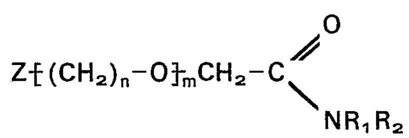

(VIII)

wherein $R_1$, $R_2$, m and n have the meaning given in Claim 1 and Z represents a halogen atom; or

(*c*) in order to prepare the amides of 2-[4-(diphenylmethylene)-1-piperidinyl]acetic acids having the general formula (I) given in Claim 1 and wherein m is 1 or 2 and Y is $-NR_1R_2$, an alkali metal salt of an ω-[4-(diphenylmethylene)-1-piperidinyl]alkanol of the formula—

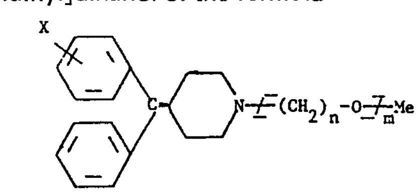

(V)

wherein X and n have the meaning given in Claim 1, m is 1 or 2 and Me represents an alkali metal, is reacted, in an inert solvent, with a 2-haloacetamide of the formula—

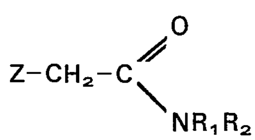

(VIII)

wherein $R_1$ and $R_2$ have the meaning given in Claim 1 and Z represents a halogen atom; or

(*d*) in order to prepare the amides of 2-[4-(diphenylmethylene)-1-piperidinyl]acetic acids

having the general formula (I), wherein Y = $-NR_1R_2$, a nitrogen compound of the formula—

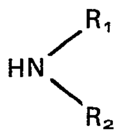

(X)

wherein $R_1$ and $R_2$ have the meaning given in Claim 1, is reacted, in an inert solvent, with a functional derivative of a 2-[4-(diphenylmethylene)-1-piperidinyl]acetic acid of the formula—

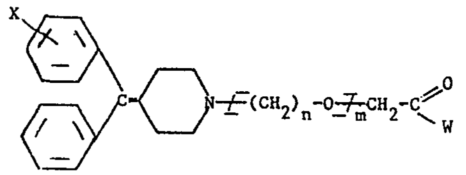

(IX)

wherein X, m and n have the meaning given in Claim 1 and W represents a halogen atom or an $-OR'$ group, wherein R' represents an alkyl radical having 1 to 4 carbon atoms; and

(*e*) optionally, the 2-[4-(diphenylmethylene)-1-piperidinyl]acetic acids or amides thus obtained are converted into their non-toxic pharmaceutically acceptable salts.

10. Therapeutic compositions containing a therapeutically effective amount of a compound of formula (I) according to Claim 1 or of a non-toxic pharmaceutically acceptable salt thereof, in association with solid or liquid pharmaceutical excipients.

**Claim** for the Contracting State: AT

Process for the preparation of 2-[4-(diphenylmethylene)-1-piperidinyl]acetic acids and their amides having the general formula—

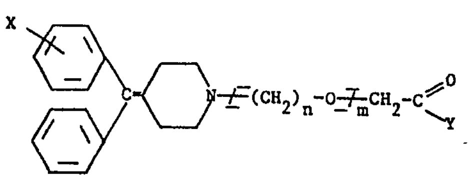

(I)

wherein

Y is an $-OH$ group or an $-NR_1R_2$ group, wherein $R_1$ and $R_2$ represent independently of each other hydrogen, an alkyl radical having 1 to 4 carbon atoms or a phenyl radical,

X represents a hydrogen atom, a halogen atom or an alkoxy radical having 1 to 4 carbon atoms,

m is 0, 1 or 2, and

n is 1 or 2,

as well as the non-toxic pharmaceutically acceptable salts thereof, characterised in that—

(*a*) in order to prepare the 2-[4-(diphenylmethylene)-1-piperidinyl]acetic acids having the general formula (I), wherein Y = OH, a functional derivative of a 2-[4-diphenylmethylene)-1-piperidinyl]acetic acid of the formula—

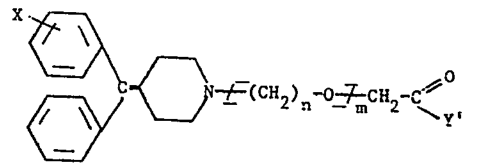

(II)

wherein X, m and n have the meaning given above and Y' represents an $-NR_1R_2$ group, $R_1$ and $R_2$ having the meaning given above, or an $-OR'$ group, wherein R' represents an alkyl radical having 1 to 4 carbon atoms, is hydrolysed, in an aqueous or hydroalcoholic medium, and with an inorganic base; or

(*b*) in order to prepare the amides of 2-[4-(diphenylmethylene)-1-piperidinyl]acetic acids having the general formula (I), wherein Y = $-NR_1R_2$, a 4-(diphenylmethylene)piperidine of the formula—

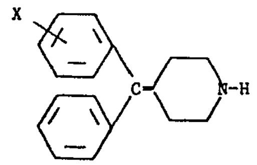

(III)

wherein X has the meaning given above, is reacted, in an inert solvent and in the presence of an acid acceptor, with an ω-haloacetamide of the formula—

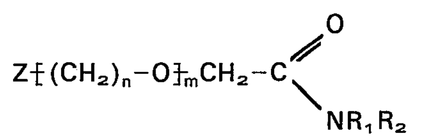

(VII)

wherein $R_1$, $R_2$, m and n have the meaning given above and Z represents a halogen atom; or

(*c*) in order to prepare the amides of 2-[4-(diphenylmethylene)-1-piperidinyl]acetic acids having the general formula (I), wherein m is 1 or 2 and Y is $-NR_1R_2$, an alkali metal salt of an ω-[4-(diphenylmethylene)-1-piperidinyl]alkanol of the formula—

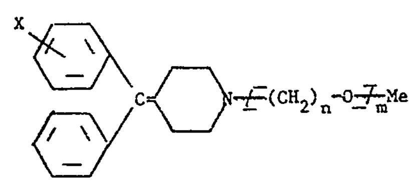

(V)

wherein X and n have the meaning given above, m is 1 or 2 and Me represents an alkali metal, is reacted, in an inert solvent, with a 2-haloacetamide of the formula—

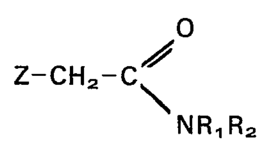

(VIII)

wherein $R_1$ and $R_2$ have the meaning given above and Z represents a halogen atom; or

(*d*) in order to prepare the amides of 2-[4-(diphenylmethylene)-1-piperidinyl]acetic acids having the general formula (I), wherein Y = $-NR_1R_2$, a nitrogen compound of the formula—

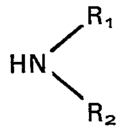

(X)

wherein $R_1$ and $R2_2$ have the meaning given above, is reacted, in an inert solvent, with a functional derivative of a 2-[4-(diphenylmethylene)-1-piperidinyl]acetic acid of the formula–

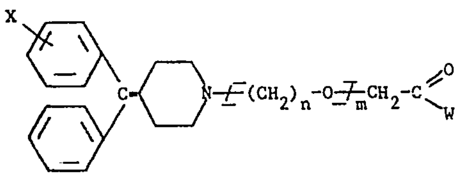

(IX)

wherein X, m and n have the meaning given above and W represents a halogen atom or an $-OR'$ group, wherein R' represents an alkyl radical having 1 to 4 carbon atoms; and

(*e*) optionally, the 2-[4-(diphenylmethylene)-1-piperidinyl]acetic acids or amides thus obtained are converted into their non-toxic pharmaceutically acceptable salts.

**Patentansprüche** für die Vertragsstaaten: BE, CH, DE, FR, GB, IT, LI, LU, NL und SE

1. 2-[4-(Diphenylmethylen)-1-piperidinyl]essigsäuren und ihre Amide entsprechend der allgemeinen Formel:

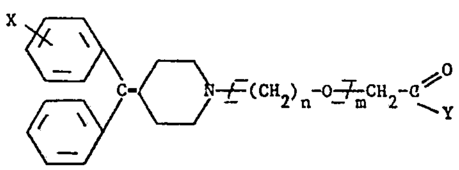

(I)

worin

Y ein $-OH$-Rest oder ein $-NR_1R_2$-Rest ist, worin $R_1$ und $R_2$ unabhängig voneinander Wasserstoff, einen Alkylrest mit 1 bis 4 Kohlenstoffatomen oder einen Phenylrest darstellen,

X ein Wasserstoffatom, ein Halogenatom oder einen Alkoxyrest mit 1 bis 4 Kohlenstoffatomen darstellt,

m 0, 1 oder 2 ist, und

n 1 oder 2 ist,

sowie deren nichttoxische, pharmazeutisch annehmbare Salze.

2. 2-[2-[2-[4-(Diphenylmethylen)-1-piperidinyl]ethoxy]ethoxy]essigsäure und ihr Hydrochlorid.

3. 2-[2-[4-(Diphenylmethylen)-1-piperidinyl]ethoxy]essigsäure und ihr Hydrochlorid.

4. 2-[2-[2-[4-(Diphenylmethylen)-1-piperidinyl]ethoxy]ethoxy]acetamid und sein Hydrochlorid.

5. 2-[2-[4-[(4-Fluorphenyl)phenylmethylen]-1-piperidinyl]ethoxy]essigsäure.

6. 2-[2-[4-[(4-Fluorphenyl)phenylmethylen]-1-piperidinyl]ethoxy]acetamid.

7. 2-[2-[4-[(4-Methoxyphenyl)phenylmethylen]-1-piperidinyl]ethoxy]acetamid und sein Hydrohylchlorid.

8. 2-[2-[4-[(2-Chlorphenyl)phenylmethylen]-1-piperidinyl]ethoxy]acetamid und sein Hydrochlorid.

9. Verfahren zur Herstellung von den in Anspruch 1 bestimmten 2-[4-(Diphenylmethylen)-1-piperidinyl]essigsäuren, ihren Amiden und ihren Salzen, dadurch gekennzeichnet, dass man:

a) um 2-[4-(Diphenylmethylen)-1-piperidinyl]essigsäuren der in Anspruch 1 gegebenen allgemeinen Formel (I) herszustellen und worin Y = OH, ein funktionelles Derivat einer 2-[4-(Diphenylmethylen)-1-piperidinyl]essigsäure der Formel:

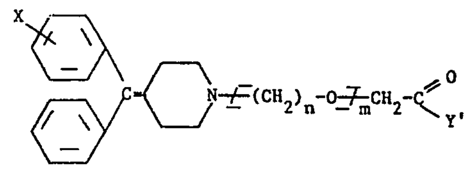

(II)

worin X, m und n die in Anspruch 1 angegebene Bedeutung haben und Y' einen $-NR_1R_2$-Rest, wobei $R_1$ und $R_2$ die in Anspruch 1 angegebene Bedeutung haben, oder einen $-OR'$-Rest darstellt, worin R' einen Alkylrest mit 1 bis 4 Kohlenstoffatomen bedeutet, in einem wässerigen oder hydroalkoholischen Mittel und mit einer anorganischen Base, hydrolysiert; oder

b) um Amide von 2-[4-(Diphenylmethylen)-1-piperidinyl]essigsäuren der in Anspruch 1 gegebenen allgemeinen Formel (I) herzustellen und worin Y = $-NR_1R_2$, ein 4-(Diphenylmethylen)-piperidin der Formel:

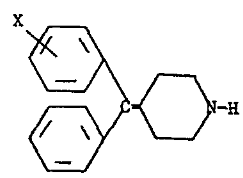

(III)

worin X die in Anspruch 1 angegebene Bedeutung hat, mit einem ω-Haloacetamid der Formel:

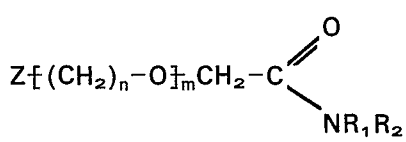

(VII)

worin $R_1$, $R_2$, m und n die in Anspruch 1 angegebene Bedeutung haben und Z ein Halogenatom darstellt, in einem inerten Lösungsmittel und in Gegenwart eines Säureakzeptors, umsetzt; oder

c) um Amide von 2-[4-(Diphenylmethylen)-1-piperidinyl]essigsäuren der in Anspruch 1 gegebenen allgemeinen Formel (I) herzustellen und worin m 1 oder 2 ist und Y = $-NR_1R_2$, ein Alkalimetallsalz eines ω-[4-(Diphenylmethylen)-1-piperidinyl]alkanols der Formel:

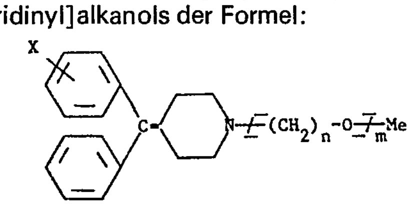

(V)

worin X und n die in Anspruch 1 angegebene Bedeutung haben, m 1 oder 2 ist und Me ein Alkalimetall darstellt, in einem inerten Lösungsmittel, mit einem 2-Haloacetamid der Formel :

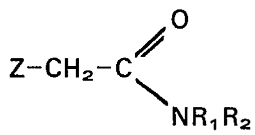
(VIII)

worin $R_1$ und $R_2$ die in Anspruch 1 angegebene Bedeutung haben und Z ein Halogenatom darstellt, umsetzt; oder

d) um Amide von 2-[4-(Diphenylmethylen)-1-piperidinyl]essigsäuren der allgemeinen Formel (I) herzustellen, worin Y = $-NR_1R_2$, eine Stickstoffverbindung der Formel:

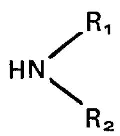
(X)

worin $R_1$ und $R_2$ die in Anspruch 1 angegebene Bedeutung haben, in einem inerten Lösungsmittel, mit einem funktionellen Derivat einer 2-[4-(Diphenylmethylen)-1-piperidinyl]essigsäure der Formel:

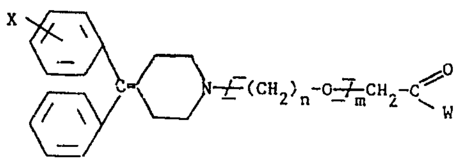
(IX)

worin X, m und n die in Anspruch 1 angegebene Bedeutung haben und W ein Halogenatom oder einen $-OR'$-Rest darstellt, worin R' einen Alkylrest mit 1 bis 4 Kohlenstoffatomen bedeutet, umsetzt; und

e) gegebenenfalls die so erhaltenen 2-[4-(Diphenylmethylen)-1-piperidinyl]essigsäuren oder Amide in ihre nichttoxischen, pharmazeutisch annehmbaren Salze umwandelt.

10. Heilmittel, enthaltend eine therapeutisch wirksame Menge einer Verbindung der in Anspruch 1 gegebenen Formel (I) oder eines nichttoxischen, pharmazeutisch annehmbaren Salzes davon in Verbindung mit festen oder flüssigen, pharmazeutischen Hilfsstoffen.

**Patentanspruch** für den Vertragsstaat: AT

Verfahren zur Herstellung von 2-[4-(Diphenylmethylen)-1-piperidinyl]essigsäuren und ihren Amiden entsprechend der allgemeinen Formel:

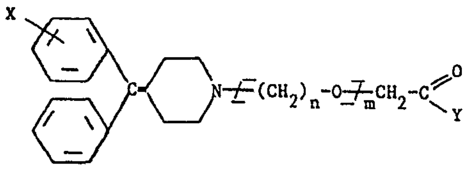
(I)

worin

Y ein $-OH$-Rest oder ein $-NR_1R_2$-Rest ist, worin $R_1$ und $R_2$ unabhängig voneinander Wasserstoff, einen Alkylrest mit 1 bis 4 Kohlenstoffatomen oder einen Phenylrest darstellen,

X ein Wasserstoffatom, ein Halogenatom oder einen Alkoxyrest mit 1 bis 4 Kohlenstoffatomen darstellt,

m 0, 1 oder 2 ist, und

n 1 oder 2 ist,

sowie von deren nichttoxischen, pharmazeutisch annehmbaren Salzen, dadurch gekennzeichnet, dass man:

a) um 2-[4-(Diphenylmethylen)-1-piperidinyl]essigsäuren der allgemeinen Formel (I) herzustellen, worin Y = OH, ein funktionelles Derivat einer 2-[4-(Diphenylmethylen)-1-piperidinyl]-essigsäure der Formel:

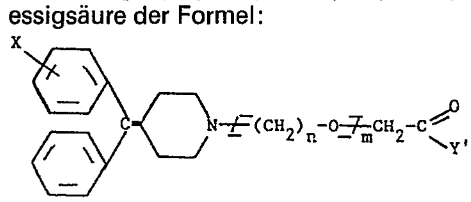
(II)

worin X, m und n die oben angegebene Bedeutung haben und Y' einen $-NR_1R_2$-Rest, wobei $R_1$ und $R_2$ die oben angegegebene Bedeutung haben, oder einen $-OR'$-Rest darstellt, worin R' einen Alkylrest mit 1 bis 4 Kohlenstoffatomen bedeutet, in einem wässerigen oder hydroalkoholischen Mittel und mit einer anorganischen Base, hydrolysiert; oder

b) um Amide von 2-[4-(Diphenylmethylen)-1-piperidinyl]essigsäuren der allgemeinen Formel (I) herzustellen, worin Y = $-NR_1R_2$, ein 4-(Diphenylmethylen)piperidin der Formel:

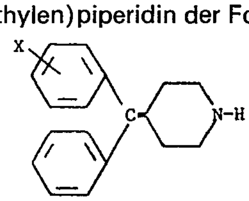
(III)

worin X die oben angegebene Bedeutung hat, mit einem ω-Haloacetamid der Formel:

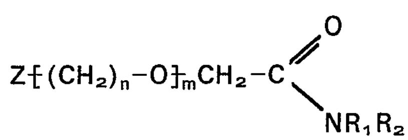
(VII)

worin $R_1$, $R_2$, m und n die oben angegebene Bedeutung haben und Z ein Halogenatom darstellt, in einem inerten Lösungsmittel und in Gegenwart eines Säureakzeptors, umsetzt; oder

c) um Amide von 2-[4-(Diphenylmethylen)-1-piperidinyl]essigsäuren der allgemeinen Formel (I) herzustellen, worin m 1 oder 2 ist und Y = $-NR_1R_2$, ein Alkalimetallsalz eines ω-[4-(Diphenylmethylen)-1-piperidinyl]alkanols der Formel:

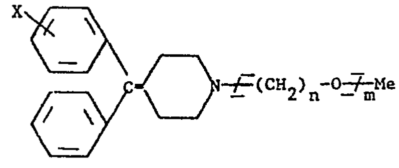
(V)

worin X und n die oben angegebene Bedeutung

haben, m 1 oder 2 ist und Me ein Alkalimetall darstellt, in einem inerten Lösungsmittel, mit einem 2-Haloacetamid der Formel:

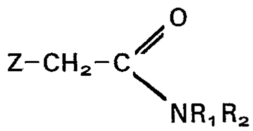

(VIII)

worin $R_1$ und $R_2$ die oben angegebene Bedeutung haben und Z ein Halogenatom darstellt, umsetzt; oder

d) um Amide von 2-[4-(Diphenylmethylen)-1-piperidinyl]essigsäuren der allgemeinen Formel (I) herzustellen, worin Y = $-NR_1R_2$, eine Stickstoffverbindung der Formel:

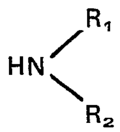

(X)

worin $R_1$ und $R_2$ die oben angegebene Bedeutung haben, i einem inerten Lösungsmittel, mit einem funktonellen Derivat einer 2-[4-(Diphenylmethylen)-1-piperidinyl]essigsäure der Formel:

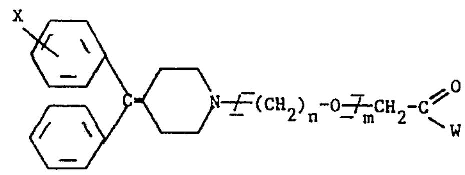

(IX)

worin X, m und n die oben angegebene Bedeutung haben und W ein Halogenatom oder einem $-OR'$-Rest darstellt, worin R' einen Alkylrest mit 1 bis 4 Kohlenstoffatomen bedeutet, umsetzt; und

e) gegebenenfalls die so erhaltenen 2-[4-(Diphenylmethylen)-1-piperidinyl]essigsäuren oder Amide in ihre nichttoxischen, pharmazeutisch annehmbaren Salze umwandelt.